# EUROPÄISCHE PATENTSCHRIFT

(11) **EP 1 074 628 B1**
(45) Veröffentlichungstag und Bekanntmachung des Hinweises auf die Patenterteilung: **15.09.2004**
(21) Anmeldenummer: 00115902.9
(22) Anmeldetag: 25.07.2000
(51) Int. Cl.: C12N 15/61, C12N 15/80, C12N 1/21, C12N 9/90, C12Q 1/68, C12P 41/00

(54) **N-Acetylaminosäureracemase**
N-Acetylamino acid racemase
Racémase d'un acide aminé acylé

(30) Priorität: 27.07.1999 DE 19935268
(43) Veröffentlichungstag der Anmeldung: 07.02.2001
(73) Patentinhaber: Degussa AG, 40474 Düsseldorf (DE)
(72) Erfinder: Verseck, Stefan, Dr., 52074 Aachen (DE); Kula, Maria-Regina, 52382 Niederzier (DE); Bommarius, Andreas, Dr., 60596 Frankfurt am Main (DE); Drauz, Karlheinz, Prof., 63579 Freigericht (DE)

(56) Entgegenhaltungen:
- EP-A- 0 304 021
- EP-A- 0 474 965
- DATABASE EMBL SEQUENCE DATABSE [Online] Hinxton, UK; 4. Oktober 1995 (1995-10-04) S. TOKUYAMA: "Amycolatopsis sp. Aaar gene for A-acetylamino acid racemase, complete cds." XP002154241
- MARSHALL C G ET AL: "Glycopeptide antibiotic resistance genes in glycopeptide-producing organisms." ANTIMICROBIAL AGENTS AND CHEMOTHERAPY, Bd. 42, Nr. 9, 1998, Seiten 2215-2220, XP002154240 ISSN: 0066-4804
- DATABASE BIOSIS [Online] BIOSCIENCES INFORMATION SERVICE, PHILADELPHIA, PA, US; 1995 MEHLING ANNETTE ET AL: "Nucleotide sequences of streptomycete 16S ribosomal DNA: Towards a specific identification system for streptomycetes using PCR." Database accession no. PREV199598547032 XP002154242 & MICROBIOLOGY (READING), Bd. 141, Nr. 9, 1995, Seiten 2139-2147, ISSN: 1350-0872

## Beschreibung

Die vorliegende Erfindung richtet sich auf eine N-Acetylaminosäureracemase (AAR) aus Amycolatopsis orientalis subspecies lurida sowie das dafür codierende Gen und das Gen enthaltende Plasmide, Vektoren und Mikroorganismen.

Mittels N-Acetylaminosäureracemasen können im Zusammenwirken mit Acylasen optisch reine Aminosäuren zu 100 % aus den entsprechenden geschützten racemischen N-Acetylaminosäuren gewonnen werden. Optisch reine Aminosäuren werden in der parenteralen Ernährung sowie zur Herstellung chiraler bioaktiver Wirkstoffe gebraucht.

Aus Streptomyces atratus Y-53 (Tokuyama et al., Appl. Microbiol. Biotechnol. 1994, 40, 835-840) und Amycolatopis sp. TS-1-60 (Tokuyama et al., Appl. Microbiol. Biotechnol. 1995a, 42, 853-859) sind bereits N-Acetylaminosäureracemasen (AAR) bekannt.

Die AAR aus Amycolatopsis sp. TS-1-60 besitzt bzgl. ihrer Aktivität eine starke Kobalt- und Manganionenabhängigkeit. Die Zugabe dieser Schwermetallionen zur Synthesebrühe ist im großtechnischen Maßstab aus Sicht des Umweltschutzes nachteilig.

Die EP 474965 ist auf die fermentative Herstellung einer Acetylaminosäureracemase aus Amycolatopsis sp. TS-1-60, neue Mikroorganismen für diese Herstellung und entsprechende DNA-Fragmente gerichtet.

In D2 (Datebase EMBL Sequence Database [Online] Hinxton, UK; 4. Oktober 1995 (1995-10-04) S. Tokuyama: Amycolatopsis sp. Aar gene for A-acetylamino acid racemase, complete cds. XP002154241) wird ein DNA-Fragment kodierend für die Aminosäuresequenz einer N-Acetylaminosäureracemase aus Amycolatopsis sp. TS-1-60 beschrieben. Desweiteren werden Vektoren, die diese Sequenz beinhalteten sowie transformierte Organismen, die diese Vektoren enthalten, beansprucht. Ebenfalls werden hier DNA-Proben und Primer, die mit diesem Fragment hybridisieren, erwähnt.

EP 304021 ist auf die Sequenzierung der 16rRNA-Gene von 17 Actinomyceten zur Erleichterung der Identifikation von Streptomyces gerichtet. Unter anderem wurde auch das 16S rRNA-Gen der Spezies Amycolatopsis orientalis ssp. lurida sequenziert.

D4 (Database Biosis [Online] Biosciences Information Service, Philadelphia, PA, US; 1995 Mehling Annette et al.: Nucleotide sequences of streptomycete 16S ribosomal DNA: Towards a specific identification system for streptomycetes using PCR. Database accession no. PREV 199598547032 OP 002154242 & Microbiology (Reading, Bd. 141, Nr. 9, 1995, Seiten 2139-2147, ISSN: 1350-0872) beschreibt die Racemisierung optisch aktiver N-Acetylaminosäuren mittels Acetylaminosäureracemase aus Amycolatopsis orientalis ssp. TS-1-60.

Aufgabe der vorliegenden Erfindung war es deshalb, eine weitere AAR zur Verfügung zu stellen, welche darüber hinaus eine geringere Aktivitätsabhängigkeit von Schwermetallionen aufweist, verglichen mit der AAR aus TS-1-60.

Gelöst wird diese Aufgabe durch eine AAR gemäß Anspruch 1. Anspruch 2 schützt die für dieses Enzym codierenden Gene. Anspruch 3 bis 5 betrifft Plasmide, Vektoren und Mikroorganismen, die diese Gene enthalten. Anspruch 6 ist auf Primer für dieses Gen gerichtet. Anspruch 7 schützt vorteilhafte Gensonden zum Aufspüren des AAR-Gens. Ansprüche 8 und 9 sind auf vorteilhafte Verwendungen der erfindungsgemäßen AAR gerichtet.

Dadurch, daß man die N-Acetylaminosäureracemase (Seq. 2) aus Amycolatopsis orientalis subspecies lurida zur Racemisierung von N-Acetylaminosäuren bereitstellt, erhält man ein Enzym, welches ausschließlich N-Acetylaminosäuren racemisiert, N-ungeschützte Aminosäuren nicht umsetzt und darüber hinaus gegenüber der AAR aus TS-1-60 eine geringere Abhängigkeit für das Schwermetallion Co²⁺ aufweist. Diese Tatsache ist für den großtechnischen Einsatz des Enzyms allerdings aus Kosten- und Umweltgesichtspunkten äußerst vorteilhaft. Die Identität auf DNA-Ebene von A. sp. TS-1-60 (Tokuyama et al., Appl. Microbiol. Biotechnol. 1995b, 42, 884-889) und A. orientalis subsp. lurida bzgl. des Gens, welches für die Racemase codiert, beträgt 86 %. Es war mithin sehr überraschend, in einer Gattung von Mikroorganismen gleiche Enzyme mit derart verschiedenen Eigenschaften zu finden.

In einer weiteren Ausgestaltung der Erfindung werden Gene (Seq. 1) codierend für die erfindungsgemäße Racemase beansprucht. Hierbei sind erfindungsgemäß auch die Gene umfaßt, welche im Rahmen der Bandbreite, die durch die Degeneration des genetischen Codes vorgegeben wird, möglich erscheinen.

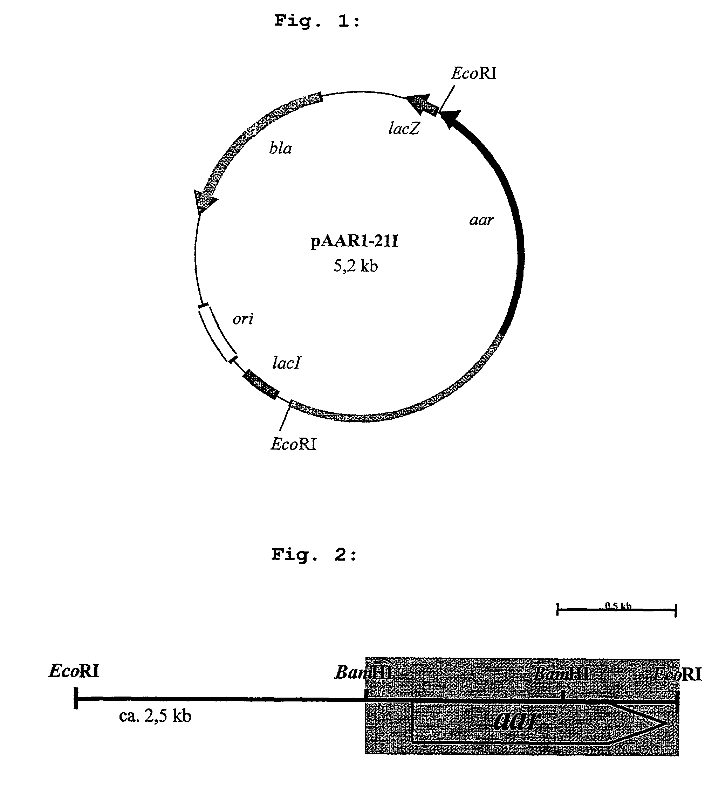

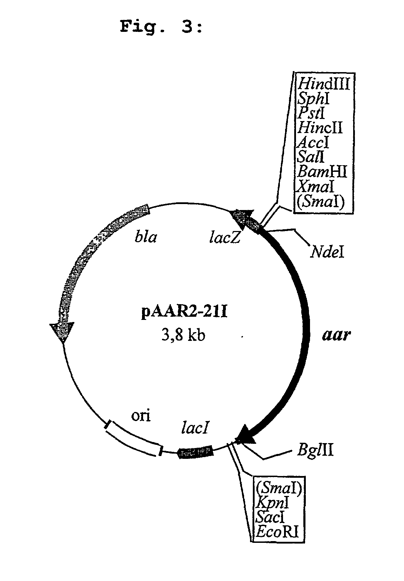

Weiterhin sind im Rahmen der Erfindung auch Plasmide oder Vektoren geschützt, welche die erfindungsgemäßen Gene aufweisen. Als bevorzugte Plasmide und Vektoren sind anzusehen pAAR1-21I, pAAR2-21I und pAAR3-21I (Fig. 1, 3 u. 4).

In einer weiteren Ausgestaltung der Erfindung sind alle Mikroorganismen, die die erfindungsgemäßen Gene aufweisen, beansprucht. Insbesondere sind dies Mikroorganismen wie die Plasmid tragenden E. coli-Stämme (DH5α bzw. BL21). Ganz besonders bevorzugt ist der Stamm DE3 in diesem Zusammenhang.

Prinzipiell kommt für die Durchführung der Erfindung jedes dem Fachmann bekannte Plasmid(Vector)/Wirts-System in Frage, in welches das Gen über eine entsprechende Schnittstelle kloniert bzw. das so entstandene Konstrukt transformiert werden kann. Dem Fachmann sind derartige Systeme geläufig, und er weiß um die Möglichkeit, daß Plasmide mit verschiedenen Wirts-Systemen kombiniert werden können. Eine Übersicht über das T7-Expressionssystem ist in Studier et al., Methods Enzymol. 1990, 185, 61-69 gegeben. Weitere geeignete Expressionssysteme können in den einschlägig bekannten Prospekten der Firmen Novagen, Promega, New England Biolabs, Clontech sowie Gibco BRL gefunden werden.

Das Ableiten geeigneter Primer geschieht durch den Vergleich von schon bekannten DNA-Sequenzen des gesuchten Gens, oder durch das "Übersetzen" von Aminosäure-Sequenzen in die Kodon-Verwendung des entsprechenden Organismus (zum Beispiel für Streptomyceten: Wright et al., Gene 1992, 113, 55-65). Auch übereinstimmende AS-Sequenzen von Proteinen aus sogenannten Superfamilien sind dabei hilfreich (Firestine et al., Chemistry & Biology 1996, 3, 779-783).

Die hier erwähnte AAR gehört dabei der Enolase-Superfamilie an (Babbitt et al., Biochemistry 1996, 35, 16589-16501). Es sind dem Fachmann deshalb bevorzugt Strategien bekannt, die zu Primeren führen, welche für den erfindungsgemäßen Zweck als vorteilhaft erscheinende Sequenz herangezogen werden können. Insbesondere können für die erfolgreiche PCRvermittelte Amplifikation eines Genabschnitts zwei Primer (AR1) und (AR5) konstruiert werden.

Weiterhin sind als bevorzugte Primer die schnittstellentragenden Anfangs- bzw. Endsequenzen des erfindungsgemäßen Gens anzusehen. Geeignete Schnittstellen sind in den oben angesprochenen Prospekten zu finden.

Ganz besonders bevorzugt sind die folgenden Sequenzen, welche die NdeI bzw. BglII-Schnittstelle aufweisen:

Mit den zwei Primern AR1 und AR5 wurde ein 504 bp großes Fragment des erfindungsgemäßen Gens mittels PCR-Technik amplifiziert. Diese Technik ist ausführlich in Saiki et al., Science (1988), 239, 487-491 gewürdigt und dem Fachmann daher bekannt. Seine Abfolge an Basenpaaren (Seq. 3) ist wie unten dargestellt:

Es diente als Teil einer Sonde zum Auffinden des beanspruchten Gens. Die Herstellung einer Gensonde aus einem Genfragment ist u. a. in Sambrook et al., A laboratory manual, Cold Spring Harbor Laboratory Press, New York (1989) dargestellt und dem Fachmann geläufig. In diesem speziellen Fall wurde das o. a. Genfragment zusammen mit der DIG-Markierung der Firma Roche Diagnostics verwendet.

Gegenstand der vorliegenden Anmeldung ist ebenfalls die Verwendung der erfindungsgemäßen Racemase in einem Prozeß zur Herstellung von optisch angereicherten Aminosäuren oder deren Derivaten. Dazu wird die racemische N-Acetylaminosäure in Gegenwart einer Acylase und der AAR unter physiologischen Bedingungen zur Reaktion gebracht. Dadurch, daß die gebildete Aminosäure dabei durch Ausfällung aus dem Gleichgewicht der Reaktion entfernt wird und die AAR aus der verbleibenden optisch angereicherten N-Acetylaminosäure immer das Racemat bildet, kommt es zur 100%igen Umsetzung des Racemats zu einer optischen Antipode der betreffenden Aminosäure.

Vorzugsweise erfolgt dieser Prozeß in einem Enzym-Membran-Reaktor (DE 1 003 110).

Mit Hilfe der oben dargestellten Sonde wurde über Southern-Hybridisierung (Sambrook et al., A laboratory manual, Cold Spring Harbor Laboratory Press, New York (1989) und Hopwood et al., A laboratory manual, The John Innes Foundation, Norwich (1985)) ein ca. 2,5 kb EcoRI Fragment aus genomischer DNA von A. orientalis subspecies lurida detektiert.

Es folgte eine Shot-gun Klonierung, in der die gesamte DNA-Population von 2,5 kb großen EcoRI Fragmenten der genomischen DNA in die zuvor mit dem Restriktionsenzym EcoRI hydrolysierten Plasmide pUC18 (Vieira et al., Gene (1982), 19, 259-268) ligiert wurden.

Die so entstandenen Vektoren wurden dann in E. coli DH5α transformiert. Die Identifikation des DNA-Fragmentes mit dem Gen der AAR erfolgte dann mittels Kolonie-Hybridisierung (Sambrook et al., A laboratory manual, Cold Spring Harbor Laboratory Press, New York (1989) und Hopwood et al., A laboratory manual, The John Innes Foundation, Norwich (1985)) unter Verwendung der zuvor beschriebenen DIGmarkierten 504 bp-Sonde.

Das erhaltene Plasmid mit dem AAR-Gen wurde pAAR1-21I (Fig. 1) genannt. Fig. 2 zeigt die Restriktionskarte des 2,5 kb EcoRI Fragmentes mit dem AAR-Gen. Das 1,3 kb-EcoRI Fragment mit dem AAR-Gen wurde doppelsträngig sequenziert und analysiert.

Für die Amplifikation des Gesamtgens mittels PCR aus pAAR1-21I wurden die Primer AR_Ex1Nde und AR_Ex2Bgl eingesetzt. Mit Hilfe dieser Primer wurde am 5-Ende des Gens eine NdeI-Restriktionsschnittstelle eingefügt und am 3'-Ende des AAR-Gens eine BglII-Schnittstelle.

Das Amplifikat wurde blunt-end in mit SmaI hydrolysiertem Plasmid pUC18 ligiert und das so entstandene Konstrukt pAAR2-21I (Fig. 3) in E. coli DH5α transformiert.

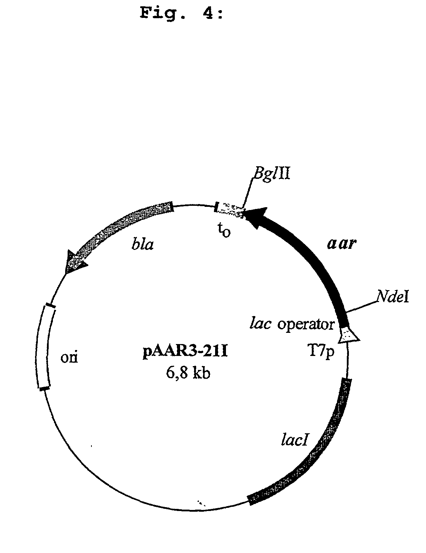

Das Einfügen dieser Restriktionsschnittstellen NdeI und BglII ermöglichte die Klonierung des Gens in den Expressionsvektor pET11a (Firma Novagen). Dieser Expressionsvektor, pAAR3-21I (Fig. 4) genannt, mit dem AAR-Gen aus A. orientalis subspecies lurida wurde in den Expressionsstamm E. coli BL21 (DE3) (Firma Novagen; enthält T7-Polymerase unter der Kontrolle des lac-Operons im Genom integriert) transformiert.

Mittels des Expressionsplasmides pAAR3-21I konnte die AAR aus A. orientalis subspecies lurida in E. coli BL21 (DE3) nach einem abgewandeltem Protokoll von Studier et al., Methods Enzymol (1990), 185, 61-89 heterolog überexprimiert werden. Die Überexpression wurde durch Isopropylthiogalactosid (IPTG) induziert. Der E. coli Expressionsstamm besaß ursprünglich keine N-Acetylaminosäureracemase-Aktivität.

Die AAR-Aktivität wurde in einem gekoppeltem enzymatischen Assay (Abb. 1) verfolgt, indem die Bildung einer deacetylierten Aminosäure aus einer N-Acetyl-D-Aminosäure, hier Methionin, mit dem HPLC-System I nachgewiesen wurde. Als Hilfsenzyme dienten die L-spezifischen Acylasen aus Schweinenieren oder Aspergillus oryzae.

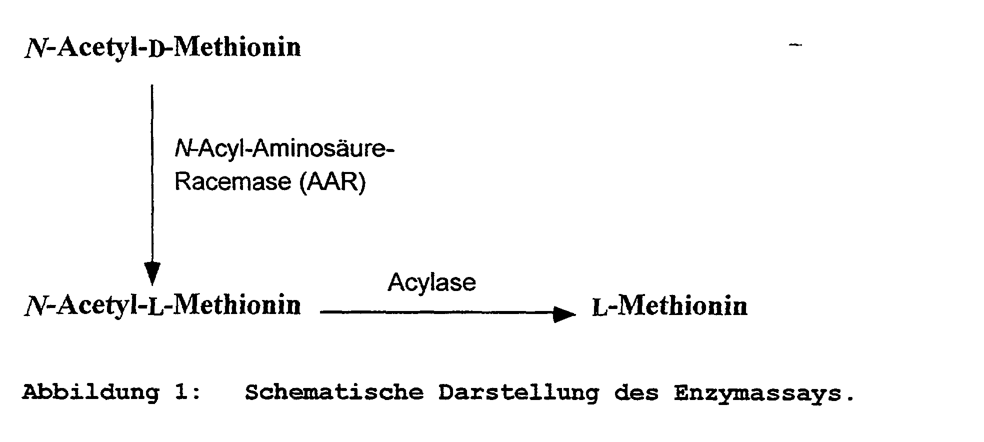

Die Aufreinigung der heterolog überexprimierten AAR erfolgte nach Zellaufschluß über 3 Schritte:
1. Hitzefällung bei 50°C für 30 min.
2. Anionen-Austauschchromatographie über Q-Sepharose (fast flow; Firma Pharmacia)
3. Hydrophobe-Interaktionschromatographie über Phenyl-Sepharose (stream line; Firma Pharmacia)

Weitere Charakterisierungen der AAR erfolgten mit dem HPLC-System II. Mit diesem Systems konnte die Aktivität der AAR direkt untersucht werden. Auf das Hilfsenzym, die Acylase, konnte so verzichtet werden, so daß störende Nebenaktivitäten vermieden werden konnten.

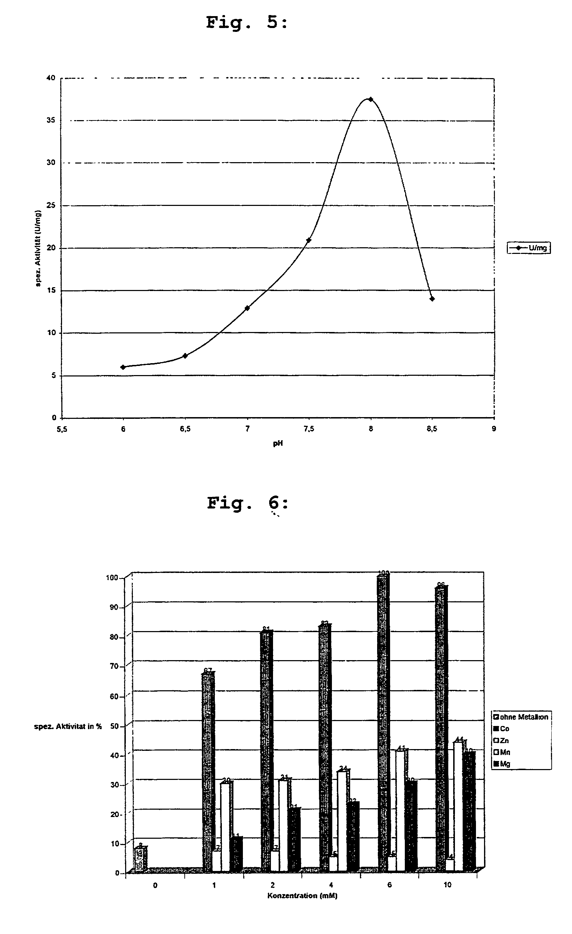

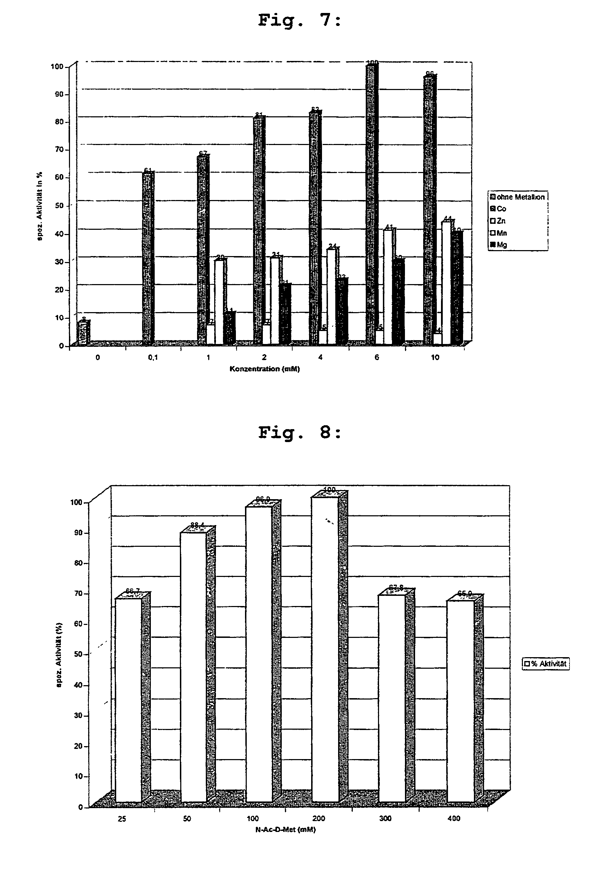

Als Eigenschaften der AAR aus A. orientalis subspecies lurida haben sich folgende herauskristallisiert:
i) Die AAR racemisiert ausschließlich N-Acetylaminosäuren, N-Acetyl-ungeschützte Aminosäuren werden nicht umgesetzt.
ii) Die überexprimierte Proteinbande der AAR erscheint in der SDS-PAGE-Analyse (Laemmli, Nature (1970), 227, 680-685), im denaturierten Zustand, bei einem Molekulargewicht von ca. 40 kDa.
iii) Die AAR besitzt ein pH-Optimum bei pH 8 (Fig. 5).
iv) Die spezifische AAR-Aktivität nach Aufreinigung betrug 30,6 U/mg mit 2 mM CoCl₂x6H₂O im Assay. Dieser Wert liegt damit ca. 30,8 % höher als der bei Tokuyama und Hatano (Appl. Microbiol. Biotechnol. 1996, 44, 774-777) für deren Racemase gefundene.
v) Die Aktivität mit 10 mM CoCl₂x6H₂O im Assay betrug 37,5 U/mg.
vi) Die Steigerung der Aktivität der AAR aus A. orientalis subspecies lurida mit 1 mM CoCl₂x6H₂O gegenüber der Aktivität ohne Metallion im Assay betrug 1250 %. Die Gabe von 1 mM CoCl₂x6H₂O im Assay bewirkte bei der AAR aus A. sp. TS-1-60 eine Steigerung von nur 496 % (Tokuyama und Hatano, Appl. Microbiol. Biotechnol. 1996, 44, 774-777).
   Daraus ergibt sich, daß die AAR aus A. orientalis subspecies lurida bei gleicher CoCl₂x6H₂O-Konzentration im Assay um 152 % aktiver ist, als die Racemase aus A. sp. TS-1-60.
vii) Weitere zweiwertige Ionen, wie MnCl₂x4H₂O, ZnSO₄x7H₂O und MgCl₂x6H₂O wurden in verschiedenen Konzentrationen im Standardenzymassay getestet. Dabei zeigten Mn und Mg noch ca. 40 % der Aktivität, bei 10 mM, im Vergleich zur Kobaltsubstitution (Fig. 6).
viii) Eine Substrat-Inhibierung tritt bei der AAR aus A. orientalis subspecies lurida bei Substratkonzentrationen von N-Acetyl-D-methionin größer 200 mM auf (Fig. 7). Für die TS-1-60 wurde eine Inhibierung schon bei 50 mM N-Acetyl-D-methionin beobachtet.

Es läßt sich festhalten, daß die vorliegende Racemase gegenüber der aus dem Stand der Technik genannten deutliche Vorteile im Hinblick auf Aktivität, Schwermetallionenabhängigkeit und Inhibierungstendenz für den Einsatz im industriellen Maßstab bereithält.

Der Mikroorganismus Amycolatopsis orientalis subsp. lurida ist unter der Nummer DSM43134 bei der Deutschen Sammlung für Mikroorganismen hinterlegt.

Unter AAR wird im Rahmen der Erfindung sowohl das native wie auch das rekombinant hergestellte Enzym verstanden.

### Beispiele:

### 1. Anzucht des Actinomyceten-Stamms Amycolatopsis orientalis subsp. lurida und Präparation der genomischen DNA

Der Actinomyceten-Stamm Amycolatopsis orientalis subsp. lurida (DSM43134) wuchs auf TSB-Medium (Oxoid, Wesel) an. Die Kultur wurde nach der Ernte zweimal mit steriler 10,3%iger Saccharoselösung gewaschen und bis zur Verwendung bei -20°C aufbewahrt. Die Präparation der genomischen DNA erfolgte nach MEHLING et al., FEMS Microbiol Lett (1995), 128, 119-126.

### 2. Oligonucleotide

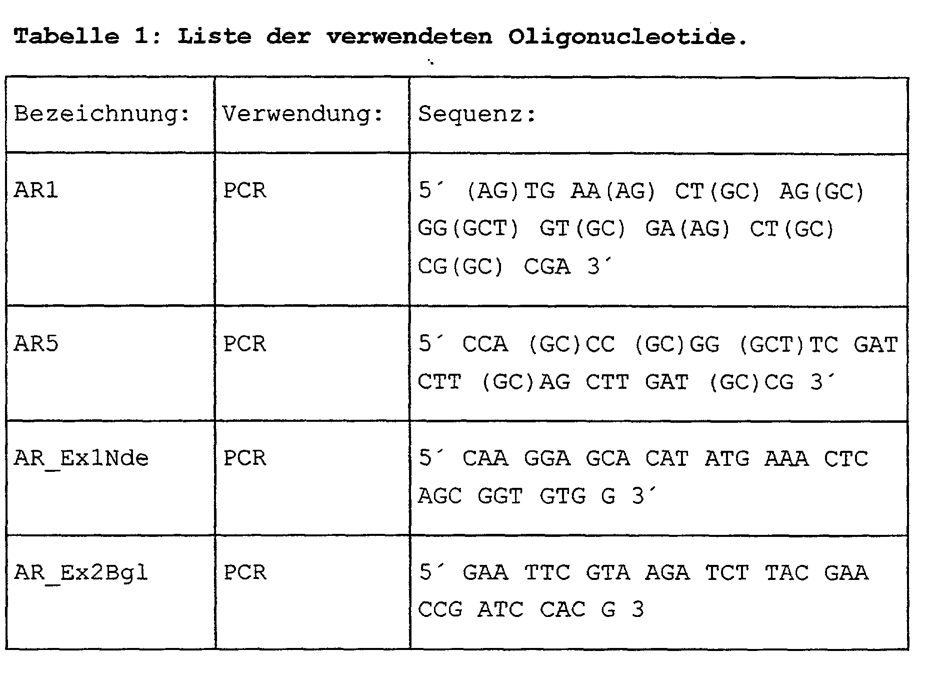

### 3. Gentechnische Methoden

Alle hier verwendeten gentechnischen Methoden sind, wenn nicht anders vermerkt, beschrieben von Sambrook et al., A laboratory manual, Cold Spring Harbor Laboratory Press, New York (1989) und Hopwood et al., A laboratory manual, The John Innes Foundation, Norwich (1985). Alle Enzyme und entsprechende Puffer wurden nach Angaben der Hersteller benutzt. Für die automatische Sequenzierung mit dem ALFred DNA-Sequencer (Pharmacia, Freiburg) wurden Cy5 markierte Primer benutzt. Der Southern-Blot und die Hybridisierung, sowie das 3'-DIG-Labeling (für nicht-radioaktiven Nachweis) der DNA-Sonden erfolgten nach Angaben der Firma Roche Diagnostics.

### 4. Polymerasekettenreaktion (PCR)

DNA-Amplifikationen mittels der Polymerasekettenreaktion wurde mit dem Biometra Thermocycler (Göttingen) in Anlehnung an Saiki et al., Science (1988), 239, 487-491 durchgeführt. Als Template diente genomische DNA aus A. orientalis subspecies lurida. Es wurde die thermostabilen DNA-Polymerase Taq (Firma Gibco BRL) in den PCR-Ansätzen eingesetzt. Die verwendeten Primerpärchen sind in der Tabelle 1 aufgelistet. Die Annealing-Temperatur A wurden über die DNA-Schmelztemperatur (Tₘ) der Oligonucleotide ermittelt. Die Zeit X für die Kettenreaktion der DNA-Polymerase richtete sich nach der 1 kb = 1 min-Regel. Alle Ansätze wurden mit ca. 50 µl Mineralöl überschichtet.

| Zusammensetzung des PCR-Ansatzes: |
|---|
| Polymerase-Puffer (10 x 5 µl) |
| dNTP's je 10 nmol |
| Primer je 50 pmol |
| DMSO 10% |
| DNA-Polymerase 1 U |
| chromosomale DANN 10 - 100 ng |
| Auffüllen mit H₂O auf 50 µl |
| (MgCl₂ nach Angaben des Herstellers der Polymerase) |

| Amplifikationsprogram: | | |
|---|---|---|
| Schritt 1 | 98°C | 5 min |
| 2 | 95°C | 1 min |
| 3 | A°C | 45 sec |
| 4 | 72°C | Xmin |
| 5 | 72°C | 2 min |

Zugabe der DNA-Polymerase bei Schritt 2, die Schritte 2 - 4 wurden 30 mal durchlaufen.

**Tabelle 2**

| **Auflistung der für die PCR verwendeten Primerpaare (vgl. Tab. 1), Annealing-Temperaturen, sowie die Länge der Amplifikate.** | | |
|---|---|---|
| Primer-Paar: | Annealing-Temperatur (Tₘ): | Länge der zu amplifizierenden DNA: |
| AR1/AR5 | 73,8°C | 1,1 kb |
| AR_Ex1Nde/AR_Ex2Bgl | 69,0°C | 1,1 kb |

### 5. Hybridisierung nach Southern und Koloniehybridisierung

Für die Hybridisierung nach Southern wurden Aliquots von Präparationen der genomischen DNA von A. orientalis subspecies lurida mit dem Restriktionsenzym EcoRI hydrolysiert. Die entstandenen DNA-Fragmente wurden dann über ein Agarose-Gel aufgetrennt. Die so aufgetrennten Fragmente wurden anschließend auf eine Nylonmembran (Hybond N⁺, Firma Amersham) geblottet. Als Sonde wurde das DIG-markierte (Firma Roche Diagnostics) 504 bp-Fragment aus A. orientalis subspecies lurida eingesetzt. Die Hybridisierungstemperatur betrug 61°C.

Das Signal gebende 2,5 kb große DNA-Fragment aus A. orientalis subspecies lurida wurde eluiert, mit dem EcoRI hydrolysiertem Plasmid pUC18 ligiert und anschließend in E. coli DH5α Shot-gun kloniert.

Die durch die Shot-gun Klonierung gewonnenen E. coli Transformanten wurden zu je 50 auf LBₐₘₚ₁₀₀-Platten ausgestrichen. Mit diesen Platten wurde dann ein Colonie-Lift und anschließend eine Kolonie-Hybridisierung durchgeführt. Als Sonde diente wiederum das DIG-markierte 504 bp Fragment aus A. orientalis subspecies lurida. Mit dieser Methode konnte eindeutig eine E. coli Transformante mit dem AAR-Gen identifiziert werden. Das Plasmid wurde pAAR1-21I genannt.

Diese Techniken sind ausführlich in Sambrook et al., A laboratory manual, Cold Spring Harbor Laboratory Press (1989) und Hopwood et al., A laboratory manual, The John Innes Foundation, Norwich (1985) gewürdigt und dem Fachmann daher bekannt.

### 6. Heterologe Expression des AAR-Enzyms aus A. orientalis subsp. lurida in E. coli BL21 (DE3)

Die standardisierte heterologe Expression des rekombinanten AAR-Enzyms aus A. orientalis subsp. lurida in E. coli BL21 (DE3) erfolgte in Anlehnung an Studier et al., Methods Enzymol (1990), 185, 61-89:

Mit Plasmiden zur Überexpression (pAAR3-21I) transformierte E. coli BL21(DE3)-Derivate wurden in LB-Medium (mit 100 µg/ml Ampicillin) über Nacht (ÜN) bei 37°C inkubiert. Es wurden dann 500 ml Hauptkultur (LB-Medium mit 100 µg/ml Ampicillin in 4 Schikane-Kolben) mit 10 ml Übernachtkultur (1 : 50) beimpft. Die T7-Polymerase wurde bei einer Zelldichte von OD₆₀₀ₙₘ = 0,5 - 0,9 mit 5 ml einer 100 mM IPTG-Lösung (Konzentration im Kolben 1 mM; IPTG = Isopropylthiogalactosid) induziert. Nach weiteren 4 - 6 h Inkubation bei 37°C wurden die Zellen geerntet.

In Rohextrakten der Expressionsklone, die in oben beschriebener Weise angezogen wurden, konnte eine AAR-Aktivität von 0,6 - 1,2 U/mg Gesamtprotein ermittelt werden.

### 7. Nachweis der Racemaseaktivität des rekombinanten AAR-Enzyms

Rohextrakte der Überexpressionsklone bzw. gereinigte Enzymfraktionen wurden in einem Enzymtest eingesetzt, indem die Bildung von L-Methionin aus N-Acetyl-D-Methionin (s. Abb. 1) über HPLC nachgewiesen werden konnte:

Der Standard-Enzymtest (abgewandelt nach Tokuyama et al., Appl Microbiol Biotechnol (1994), 40, 835-840; ibid, Appl Microbiol Biotechnol (1995a), 42, 853-859 setzte sich wie folgt zusammen:
Lösungsmittel: Tris/HCl, pH 7,5 50 mM
N-Acetyl-D-Methionin 25 mM
Cobaltchlorid 2 mM
Acylase I (ASch o. AAsp) 1 U
Protein 2 - 150 µg aufgereinigtes Protein o. Gesamtprotein Endvolumen 200 µl

Acylase I und Co²⁺ liegen dabei im Überschuß vor, so daß die AAR-Reaktion der geschwindigkeitsbestimmende Schritt ist. Die Inkubation erfolgte 10-40 min bei 30°C. Die Reaktion wurde dann durch 3minütiges Kochen gestoppt. Die Analyse der Reaktionsprodukte erfolgte mittels HPLC (System I).

Wenn nicht anders erwähnt, wurde Acylase I aus Schweinenieren (ASch) im Enzymtest eingesetzt und als Ion Co²⁺ (CoCl₂x6H₂O). Alternativ wurde aber auch Acylase I aus Aspergillus oryzae (AAsp) verwendet.

In Assays, welche über HPLC-System II analysiert wurden, konnte die Acylase weggelassen werden, da mit diesem System N-Acetylaminosäuren direkt enantioselektiv getrennt und nachgewiesen werden konnten.

Außer Co²⁺ (CoCl₂ x 6H₂O) wurden auch andere zweiwertige Ionen, wie MnCl₂ x 4H₂O, zu SO₄ x 7H₂O und MgCl₂ x 6H₂O in verschiedenen Konzentrationen eingesetzt (0,1-10 mM).

Die Substratinhibition wurde getestet, indem die Aktivität der AAR mit 25 bis 400 mM N-Acetyl-D-methionin als Substrat versetzt wurde.

### 8. HPLC-Analyse

### System I:

Säule: RP C18, 5 µm, 250 x 4,6 mm, Chromasil®
Laufmittel A: 23 mM Natriumacetat, 10% Acetonitril, pH 6,0
Laufmittel B: 100% Acetonitril
Flußrate: 1 ml/min
Probenvolumen: 20 µl
Detektion: UV-VIS 225 nm
Fluoreszens: 340/440 nm
Derivatiserung: auf Basis o-Phthaldialdehyd (OPA)/N-Isobutyryl-L-Cystein (IBLC) nach Brückner et al., Journal of Chromatography A (1994), 666, 259-273.

| Gradient: | |
|---|---|
| Zeit | Gemisch |
| 0 min | 0% B |
| 20 min | 24% B |
| 22 min | 100% B |
| 23 min | 100% B |
| 25 min | 0% B |
| 35 min | 0% B |

### System II:

### Säule: ENAN 1, Merget, 145 x 4,6 mm, (Dr. K. Günther, Degussa-Hüls AG, persönliche Leihgabe)

Laufmittel A: 700 ml Methanol, 300 ml Ammoniumacetat (0,01 M), 0,5 ml Eisessig
Flußrate: 1 ml/min
Probenvolumen: 20 µl
Detektion: UV-VIS 225 nm
Gradient: isokratisch

### 9. Aufreinigung der AAR aus A. orientalis subspecies lurida

Die Aufreinigung der heterolog überexprimierten AAR erfolgte nach Zellaufschluß über 3 Schritte:
1. Zellaufschluß: 30%ige Zellsuspension mit Tris/HCl (pH 7,5) und das 1,5fache an Glasperlen (Durchmesser 0,3 mm) wurden vermengt und im Disintegrator S (für 2 mal 15 min) aufgeschlossen.
2. Hitzefällung bei 50°C für 30 min.
3. Anionen-Austauschchromatographie über Q-Sepharose (fast flow; Firma Pharmacia), Elution bei ca. 25 % Laufmittel B.
4. Hydrophobe-Interaktionschromatographie über Phenyl-Sepharose (stream line; Firma Pharmacia), Elution bei ca. 40 % Laufmittel B.

Die Elution erfolgte jeweils mit 50 mM Tris/HCl, pH 7,5 (Laufmittel A) über einen linearen Gradienten mit 0,5 M (NH₄)₂SO₄ in 50 mM Tris/HCl, pH 7,5 (Laufmittel B).

## Patentansprüche

1. N-Acetylaminosäureracemase aus Amycolatopsis orientalis subspecies lurida gemäß Seq. ID Nr. 2.

2. Gene codierend für die Racemase gemäß Anspruch 1.

3. Plasmid aufweisend die Gene nach Anspruch 2.

4. Vektor aufweisend die Gene nach Anspruch 2.

5. E. coli aufweisend die Gene nach Anspruch 2.

6. Primer für ein Gen nach Anspruch 2 gemäß den Seq. ID Nrs. 4-7.

7. Sonde für ein Gen nach Anspruch 2 gemäß der Seq. ID Nrs. 3.

8. Verwendung der Racemase nach Anspruch 1 in einem Prozeß zur Herstellung von enantiomer angereicherten Aminosäuren oder deren Derivaten.

9. Verwendung nach Anspruch 8,
**dadurch gekennzeichnet, daß**
man den Prozeß in einem Enzym-Membran-Reaktor durchführt.

## Claims

1. N-Acetyl amino acid racemase from Amycolatopsis orientalis subspecies lurida according to SEQ ID no. 2.

2. Genes coding for the racemase according to claim 1.

3. A plasmid comprising the genes according to claim 2.

4. A vector comprising the genes according to claim 2.

5. E. coli comprising the genes according to claim 2.

6. A primer for a gene according to claim 2 according to SEQ ID nos. 4-7.

7. A probe for a gene according to claim 2 according to SEQ ID no. 3.

8. Use of the racemase according to claim 1 in a process for the production of enantiomerically enriched amino acids or the derivatives thereof.

9. Use according to claim 8,
**characterised in that**
the process is performed in an enzyme membrane reactor.

## Revendications

1. Racémase de N-acétylaminoacides issue d'amycolatopsis orientalis sous-espèce lurida selon la Séq. ID n° 2.

2. Gène codant pour la racémase selon la revendication 1.

3. Plasmide présentant le gène selon la revendication 2.

4. Vecteur présentant le gène selon la revendication 2.

5. E.Coli présentant le gène selon la revendication 2.

6. Primer pour un gène selon la revendication 2, conforme aux Séq. ID n° 4-7.

7. Sonde pour un gène selon la revendication 2, conforme à la Séq. ID n° 3.

8. Utilisation de racémase selon la revendication 1, dans un procédé pour la préparation d'aminoacides enrichis d'énantiomères ou leurs dérivés.

9. Utilisation selon la revendication 8,
**caractérisée en ce que**
le procédé est effectué dans un réacteur à membrane d'enzyme.


### SEQUENZPROTOKOLL

<110> Degussa-Huels Aktiengesellschaft
<120> N-Acetylaminosäureracemase
<130> 990095 AM
<140>
   <141>
<160> 7
<170> PatentIn Ver. 2.1
<210> 1
   <211> 1107
   <212> DNA
   <213> Amycolatopsis orientalis
<220>
   <221> CDS
   <222> (1)..(1107)
<400> 1
   gtg aaa ctc agc ggt gtg gaa ctg cgc cgg gtc cgg atg ccg ctc gtg    48
   Val Lys Leu Ser Gly Val Glu Leu Arg Arg Val Arg Met Pro Leu Val
     1               5                  10                  15
   gcc ccg ttc cgg acg tcg ttc ggg acg cag tcc gag cgg gaa ttg ctg    96
   Ala Pro Phe Arg Thr Ser Phe Gly Thr Gln Ser Glu Arg Glu Leu Leu
                20                  25                  30
   ctg gtc cgc gcg gtg acc ccg gcg ggc gag ggc tgg ggc gaa tgt gtc    144
   Leu Val Arg Ala Val Thr Pro Ala Gly Glu Gly Trp Gly Glu Cys Val
            35                  40                  45
   gcg atg gag gcg ccg ctc tac tcg tcg gag tac aac gac gcc gcc gag    192
   Ala Met Glu Ala Pro Leu Tyr Ser Ser Glu Tyr Asn Asp Ala Ala Glu
        50                  55                  60
   cac gtg ctg cgg aac cat ctg atc ccc gca ctg ctg gcg gcc gag gac    240
   His Val Leu Arg Asn His Leu Ile Pro Ala Leu Leu Ala Ala Glu Asp
    65                  70                  75                  80
   gtg acc gcg cac aag gtg acg ccg ttg ctg gcg aag ttc aag ggc cac    288
   Val Thr Ala His Lys Val Thr Pro Leu Leu Ala Lys Phe Lys Gly His
                    85                  90                  95
   cgg atg gcg aag ggc gcg ctg gag atg gcg gtc ctc gac gcc gaa ctc    336
   Arg Met Ala Lys Gly Ala Leu Glu Met Ala Val Leu Asp Ala Glu Leu
               100                 105                 110
   cgc gcg cat gac cgg tcc ttc gcg gcc gag ctg ggg tcc act cgc gac    384
   Arg Ala His Asp Arg Ser Phe Ala Ala Glu Leu Gly Ser Thr Arg Asp
           115                 120                 125
   tcc gtg gcc tgc ggg gtc tcg gtc ggg atc atg gac tcg atc ccg cac    432
   Ser Val Ala Cys Gly Val Ser Val Gly Ile Met Asp Ser Ile Pro His
               130                 135                 140
   ctg ctc gac gtc gtc ggc ggc tac ctc gac gag ggc tac gtc cgg atc     480
   Leu Leu Asp Val Val Gly Gly Tyr Leu Asp Glu Gly Tyr Val Arg Ile
   145                 150                 155                 160
   aag ctg aag atc gag ccc ggc tgg gac gtc gag ccg gtc cgg cag gtg     528
   Lys Leu Lys Ile Glu Pro Gly Trp Asp Val Glu Pro Val Arg Gln Val
                   165                 170                 175
   cgt gag cgc ttc ggt gac gac gtg ctg ctg cag gtc gac gcg aac acc     576
   Arg Glu Arg Phe Gly Asp Asp Val Leu Leu Gln Val Asp Ala Asn Thr
               180                 185                 190
   gcg tac acg ctg ggc gac gcg ccc ctg ctg tcc cgg ctc gac ccg ttc     624
   Ala Tyr Thr Leu Gly Asp Ala Pro Leu Leu Ser Arg Leu Asp Pro Phe
           195                 200                 205
   gac ctg ctg ctg atc gag cag ccg ctc gaa gaa gag gac gtg ctc ggc     672
   Asp Leu Leu Leu Ile Glu Gln Pro Leu Glu Glu Glu Asp Val Leu Gly
       210                 215                 220
   cac gcc gag ctg gcc aag cgg atc cgg acg ccg atc tgc ctc gac gag     720
   His Ala Glu Leu Ala Lys Arg Ile Arg Thr Pro Ile Cys Leu Asp Glu
   225                 230                 235                 240
   tcg atc gtc tcg gcc aag gcc gcc gcg gac gcg atc aag ctc ggc gcc     768
   Ser Ile Val Ser Ala Lys Ala Ala Ala Asp Ala Ile Lys Leu Gly Ala
                   245                 250                 255
   tgc cag atc gtc aac atc aaa ccg ggc cgg gtc ggc gga tac ctc gaa     816
   Cys Gln Ile Val Asn Ile Lys Pro Gly Arg Val Gly Gly Tyr Leu Glu
               260                 265                 270
   gcc cgc cgg gtg cac gac gtc tgc gcg gca cac ggg atc gcg gtg tgg     864
   Ala Arg Arg Val His Asp Val Cys Ala Ala His Gly Ile Ala Val Trp
           275                 280                 285
   tgc ggc ggg atg atc gag acc ggg ctc ggc cgg gcg gcc aac gtc gca     912
   Cys Gly Gly Met Ile Glu Thr Gly Leu Gly Arg Ala Ala Asn Val Ala
       290                 295                 300
   ctg gcc tcg ctg ccc ggc ttc acg ctg ccg ggg gac acc tcg gcg tcc     960
   Leu Ala Ser Leu Pro Gly Phe Thr Leu Pro Gly Asp Thr Ser Ala Ser
   305                 310                 315                 320
   ggc cgg ttc tat cgc acc gac atc acc gag ccg ttc gtg ctg gac gcc    1008
   Gly Arg Phe Tyr Arg Thr Asp Ile Thr Glu Pro Phe Val Leu Asp Ala
                   325                 330                 335
   ggg cat ctg ccg gtg ccg acc ggg ccg ggc ctc ggg gtg act ccg att    1056
   Gly His Leu Pro Val Pro Thr Gly Pro Gly Leu Gly Val Thr Pro Ile
               340                 345                 350
   ccg gat ctt ctg gac gag gtc acc acg gag aaa gcg tgg atc ggt tcg    1104
   Pro Asp Leu Leu Asp Glu Val Thr Thr Glu Lys Ala Trp Ile Gly Ser
           355                 360                 365
   tag                                                                1107
<210> 2
   <211> 369
   <212> PRT
   <213> Amycolatopsis orientalis
<400> 2
<210> 3
   <211> 504
   <212> DNA
   <213> Künstliche Sequenz
<220>
   <223> Beschreibung der künstlichen Sequenz: Gensonde
<400> 3
   atgaaactga gcggcgtgga actgcggcga gtccggatgc cgctcgtggc cccgttccgg 60
   acgtcgttcg ggacgcagtc cgagcgggaa ttgctgctgg tccgcgcggt gaccccggcg 120
   ggcgagggct ggggcgaatg tgtcgcgatg gaggcgccgc tctactcgtc ggagtacaac 180
   gacgccgccg agcacgtgct gcggaaccat ctgatccccg cactgctggc ggccgaggac 240
   gtgaccgcgc acaaggtgac gccgttgctg gcgaagttca agggccaccg gatggcgaag 300
   ggcgcgctgg agatggcggt cctcgacgcc gaactccgcg cgcatgaccg gtccttcgcg 360
   gccgagctgg ggtccactcg cgactccgtg gcctgcgggg tctcggtcgg gatcatggac 420
   tcgatcccgc acctgctcga cgtcgtcggc ggctacctcg acgagggcta cgtccgcatc 480
   aagctcaaga tcgaacccgg ctgg                                        504
<210> 4
   <211> 30
   <212> DNA
   <213> Künstliche Sequenz
<220>
   <221> primer_bind
   <222> (1)..(30)
<220>
   <223> Beschreibung der künstlichen Sequenz: Primer AR1
<400> 4
   atgaaactga gcggcgtgga actgcggcga 30
<210> 5
   <211> 30
   <212> DNA
   <213> Künstliche Sequenz
<220>
   <221> primer_bind
   <222> (1)..(30)
<220>
   <223> Beschreibung der künstlichen Sequenz: Primer AR5
<400> 5
   cgcatcaagc tcaagatcga acccggctgg 30
<210> 6
   <211> 31
   <212> DNA
   <213> Künstliche Sequenz
<220>
   <223> Beschreibung der künstlichen Sequenz: Primer AR_EX2Bgl
<400> 6
   gaattcgtaa gatcttacga accgatccac g 31
<210> 7
   <211> 31
   <212> DNA
   <213> Künstliche Sequenz
<220>
   <223> Beschreibung der künstlichen Sequenz: Primer AR_EX1Nde
<400> 7
   caaggagcac atatgaaact cagcggtgtg g 31